# EUROPÄISCHE PATENTSCHRIFT

(11) **EP 0 791 047 B1**
(45) Veröffentlichungstag und Bekanntmachung des Hinweises auf die Patenterteilung: **23.12.1998**
(21) Anmeldenummer: 95936568.5
(22) Anmeldetag: 30.10.1995
(51) Int. Cl.: C11D 17/00, A61L 9/01

(54) **REINIGUNGSMITTELSTÜCK FÜR SPÜLTOILETTEN**
FLUSH TOILET CLEANSERS IN BAR FORM
ELEMENT DETERGENT POUR TOILETTES A CHASSE D'EAU

(30) Priorität: 07.11.1994 DE 4439677
(43) Veröffentlichungstag der Anmeldung: 27.08.1997
(73) Patentinhaber: Henkel Kommanditgesellschaft auf Aktien, 40191 Düsseldorf (DE)
(72) Erfinder: MENKE, Ronald, D-40822 Mettmann (DE); DITZE, Alexander, D-42859 Remscheid (DE); PRAUS, Gerd, D-47807 Krefeld (DE)
(86) Internationale Anmeldenummer: EP9504245
(87) Internationale Veröffentlichungsnummer: WO9614392

(56) Entgegenhaltungen:
- EP-A- 0 055 100
- DE-A- 3 225 292
- US-A- 3 856 932
- DATABASE WPI Section Ch, Week 8712 Derwent Publications Ltd., London, GB; Class D25, AN 87-084278 XP002000048 & JP,A,62 036 500 (KAO CORP) , 17.Februar 1987
- DATABASE WPI Section Ch, Week 9525 Derwent Publications Ltd., London, GB; Class D25, AN 95-190984 XP002000049 & JP,A,07 109 499 (OSAKA SEIYAKU KK) , 25.April 1995
- DATABASE WPI Section Ch, Week 8346 Derwent Publications Ltd., London, GB; Class A97, AN 83-816702 XP002000050 & JP,A,58 168 699 (JAPAN SYNTHETIC RUBBER) , 5.Oktober 1983
- DATABASE WPI Section Ch, Week 8836 Derwent Publications Ltd., London, GB; Class A97, AN 88-252873 XP002000051 & JP,A,63 182 400 (OKUDA YAKUHIN SHOKA) , 27.Juli 1988
- DATABASE WPI Section Ch, Week 8705 Derwent Publications Ltd., London, GB; Class A97, AN 87-034120 XP002000052 & JP,A,61 291 695 (KAO CORP) , 22.Dezember 1986

## Beschreibung

Die vorliegende Erfindung liegt auf dem Gebiet der stückförmigen Reinigungsmittel und betrifft solche Mittel, die bei der Reinigung und Desinfektion von Spültoiletten eingesetzt werden.

Reinigungsmittel in Stückform werden zur Toilettenreinigung vor allem deshalb eingesetzt, weil es mit ihrer Hilfe möglich ist, eine weitgehend automatische Reinhaltung der Toiletten zu erreichen. Die Reinigungsmittelstücke werden entweder in den Wasserkasten von Spültoiletten eingelegt, wo sie sich allmählich auflösen und die reinigenden Wirkstoffe an das Spülwasser abgeben, oder sie werden in der Toilettenschüssel so angebracht, daß sie nur bei jedem Spülvorgang vom Spülwasser überströmt werden und während dieser kurzen Zeit die reinigungsaktiven Wirkstoffe freisetzen. Üblicherweise enthalten die Reinigungsmittelstücke zusätzlich Parfüm zur Raumbeduftung. Für die Zusammensetzung derartiger Reinigungsmittelstücke sind zahlreiche Vorschläge gemacht worden, von denen hier nur Vorschläge genannt werden sollen, die sich mit mehrteiligen Reinigungsmittelstücken befassen (EP 55 100, EP 101 402). Die mehrteilige Form wurde vorgeschlagen, weil sie es ermöglicht, chemisch miteinander unverträgliche Wirkstoffe oder Bestandteile in einem Stück unterzubringen, ohne daß es während der Lagerung zu einer Wechselwirkung zwischen diesen Bestandteilen kommt. Allen vorbekannten Reinigungsmittelstücken ist gemeinsam, daß sie beim Gebrauch allmählich kleiner werden und sich ihre Oberfläche dabei stetig verringert. Andererseits hängt die Menge an Parfüm und anderen Wirksubstanzen, die pro Zeiteinheit an die Umgebung oder das vorbeifließende Wasser abgegeben werden, unmittelbar von der zur Verfügung stehenden Oberfläche des Reinigungsmittelstücks ab. Demzufolge wird die pro Zeiteinheit abgegebene Menge an Parfüm und anderen Wirkstoffen im Verlaufe des Gebrauchs der Reingungsmittelstücke ebenfalls immer geringer. Um gegen Ende der Gebrauchszeit dieser Reingungsmittelstücke noch eine ausreichende Menge Wirkstoff freizusetzen, mußte die Zusammensetzung der Mittel deshalb so gewählt werden, daß zu Anfang weitaus mehr als die notwendige Menge an Wirkstoff pro Zeiteinheit abgegeben wurde. Während dies bei reinigend wirkenden Komponenten der Stücke vom Verbraucher meist hingenommen wurde, wurde eine Überdosierung an Parfüm häufig als störend empfunden. Aber auch die Überdosierung reinigend wirkender Stoffe ist aus ökologischen Gründen bedenklich. Eine technisch einfache Lösung für dieses Problem der gleichmäßigen Wirkstoffabgabe über den gesamten Gebrauchszeitraum ist bisher nicht vorgeschlagen worden.

Die vorliegende Erfindung bietet nun eine Lösung dieses Problems in Form eines Reinigungsmittelstücks, das aus wenigstens zwei unterschiedlich zusammengesetzten Massen besteht, wobei eine der Massen von der oder den anderen Massen wenigstens teilweise umschlossen wird und die umschlossene Masse und wenigstens eine der anderen Massen wenigstens einen gleichen Wirkstoff enthalten, dessen Konzentration in der umschlossenen Masse wenigstens das 1,3fache der Konzentration des gleichen Wirkstoffs in der oder den umschließenden Massen beträgt und der Gehalt an Salzen langkettiger Fettsäuren in den Massen nicht mehr als 25 Gew.-% beträgt. Vorzugsweise ist die Konzentration dieses Wirkstoffs in der umschlossenen Masse 2- bis 10mal so hoch wie in einer der umschließenden Massen.

Durch die besondere Gestaltung der neuen stückförmigen Reinigungsmittel steht zu Beginn des Gebrauchs für die Abgabe des Wirkstoffs an die Umgebung oder das vorbeifließende Wasser zunächst allein oder überwiegend die Oberfläche der Masse zur Verfügung, die den Wirkstoff in geringerer Konzentration enthält. Im Laufe des Gebrauchs wird diese Masse abgetragen und die Oberfläche der bisher umschlossenen Masse freigelegt, so daß dann auch aus dieser Masse Wirkstoff abgegeben werden kann. Da in dieser umschlossenen Masse die Konzentration des Wirkstoffs deutlich höher ist als in der umschließenden Masse, nimmt die pro Zeiteinheit abgegebene Menge an Wirkstoff wieder zu, so daß die durch die Verringerung der Oberfläche bedingte Abnahme der Wirkstoffabgabe mehr oder weniger ausgeglichen oder gar überkompensiert wird. In jedem Falle ist mit den erfindungsgemäßen Reinigungsmittelstücken eine wesentlich gleichmäßigere Abgabegeschwindigkeit des Wirkstoffs über die gesamte Gebrauchsdauer des Stückes als bei herkömmlichen Stücken zu erreichen. Eine Feinsteuerung der Abgabecharakteristik ist über die Konzentrationsverhältnisse des Wirkstoffs in den einzelnen Massen und über die geometrische Gestaltung und Anordnung der Massen zueinander möglich.

Im Sinne der vorliegenden Erfindung ist die Masse, die den Wirkstoff in der höheren Konzentration enthält, dann wenigstens teilweise umschlossen, wenn wenigstens 50 % der Oberfläche des aus dieser Masse gebildeten Teilstücks innerhalb des gesamten Reinigungsmittelstücks von den anderen Massen bedeckt sind, so daß dieser Anteil der Oberfläche nicht für die Abgabe des Wirkstoffs zu Beginn des Gebrauchs zur Verfügung steht. Vorzugsweise sollen wenigstens 70 %, insbesondere wenigstens 80 % der Oberfläche von den anderen Massen bedeckt sein. Im Extremfall kann die Masse, die den Wirkstoff in der höheren Konzentration enthält, vollständig von den anderen Massen umschlossen sein. Vorzugsweise ist die umschlossene (innere) Masse nur von einer weiteren Masse umschlossen.

Für die Herstellung der Reinigungsmittelstücke sind verschiedenste Verfahren brauchbar. So ist es möglich, die einzelnen Teilstücke, die aus den unterschiedlichen Massen bestehen, zunächst getrennt herzustellen und dann beispielsweise unter Druck, gegebenfalls unter gleichzeitiger Verformung, zusammenzupressen, wobei auch Bindemittel eingesetzt werden können. Denkbar ist aber auch, zunächst nur eine oder einige der Massen auszuformen und die anderen Massen in flüssiger Form hinzuzufügen, wobei das endgültige Reinigungsmittelstück in einem Gießvorgang unter anschließender Erstarrung entsteht. Ein bevorzugtes Herstellungsverfahren für die erfindungsgemäßen Reinigungsmittelstücke ist das Extrusionsverfahren, bei dem die einzelnen Massen getrennt aufgemischt und extrudiert werden, wobei die austretenden Stränge so geformt und zusammengefügt werden, daß nach dem Schneiden der zusammengefügten Stränge unmittelbar die gewünschten Reinigungsmittelstücke entstehen. Ganz besonders bevorzugt wird dabei ein Extrusionsverfahren, bei dem die Masse, die den Wirkstoff in der höheren Konzentration enthält, in einer Zwei- oder Mehrstoffdüse als Kernstrang innerhalb eines mantelförmig diesen Kern umgebenden Stranges aus der Masse, die den Wirkstoff in geringerer Konzentration enthält, extrudiert wird. Dieses Verfahren kann so geführt werden, daß die Stränge koaxial austreten, doch kann es auch vorteilhaft sein, wenn der Kernstrang nicht in der Mitte der den Mantel bildenden Masse placiert ist. Die letztere Form erlaubt nämlich die Herstellung von Stücken, bei denen die Oberfläche der umschlossenen Masse im Laufe des Gebrauchs nicht sprunghaft, sondern allmählich vergrößert wird.

In den Zeichnungen sind einige typische Anordnungen der Masseteile in den Reinigungsmittelstücken näher demonstriert. Während in den Abbildungen 1 bis 7 Reinigungsmittelstücke wiedergegeben sind, die aus zwei Masseteilen bestehen, setzen sich die Reinigungsmittelstücke der Abbildungen 8 und 9 aus drei Masseteilen zusammen.

In einer bevorzugten Ausführungsform steht die umschließende Masse zur umschlossenen Masse im Gewichtsverhältnis von 1 : 1 bis 2 : 1.

Die Zusammensetzung der einzelnen Massen, die zusammen die erfindungsgemäßen Reinigungsmittelstücke bilden, kann bis auf die unterschiedlichen Gehalte an Wirkstoff im wesentlichen gleich sein, doch ist dies keine Voraussetzung für die erfindungsgemäß erreichbaren Effekte. Diese lassen sich auch dann erzielen, wenn die einzelnen Massen deutlich voneinander abweichende Zusammensetzung aufweisen. Es ist daher möglich, die Zusammensetzung der einzelnen Massen weitgehend auch den sonstigen Forderungen, die sich beispielsweise im Hinblick auf Herstellbarkeit oder Lagerung ergeben, anzupassen. Dabei können zur Herstellung der Massen alle Substanzen verwendet werden, die auch bisher zur Herstellung von Reinigungsmittelstücken für Toiletten verwendet wurden. Zu diesen Substanzen zählen insbesondere Tenside, Desinfektions- und Bleichmittel, Enzyme, Salze, Säuren, Komplexbildner, Füllstoffe, Farbstoffe, Parfüm, Abspülregulatoren und Plastifikatoren. Als Wirkstoffe im Sinne dieser Erfindung werden dabei Tenside, Desinfektionsmittel, Bleichmittel, Enzyme, Säuren, Komplexbildner, Farbstoffe und Duftstoffe angesehen, während Substanzen wie Abspülregulatoren und Plastifikatoren, Salze und Füllstoffe, die im wesentlichen nur als Konsistenzgeber und Moderatoren für die Abspülgeschwindigkeit dienen, in diesem Sinne nicht als Wirkstoffe gelten. Als bevorzugte Wirkstoffe sind im Sinne der vorliegenden Erfindung Parfüm, Komplexbildner, Säuren, Desinfektionsmittel, Bleichmittel und Enzyme zu nennen, von denen wiederum Parfüm ganz besonders bevorzugt wird. Sofern in den Reinigungsmittelstücken mehrere Substanzen, die als Wirkstoffe gelten, enthalten sind, genügt es im Sinne dieser Erfindung, wenn einer dieser Wirkstoffe die oben genannte Bedingung, daß er in der umschlossenen Masse in höherer Konzentration enthalten ist, erfüllt.

Zur Verwendung in den erfindungsgemäßen Mitteln können Tenside aus allen bekannten Klassen, nämlich anionische, nichtionische, kationische und amphotere Tenside ausgewählt werden, doch werden vorzugsweise anionische und/oder nichtionische Tenside verwendet.

Als anionische Tenside finden insbesondere solche vom Sulfat- und Sulfonattyp Verwendung, doch können auch andere Typen wie Salze von Fettsäurecyanamiden oder Salze von Alkylethercarbonsäuren, wie sie aus ethoxilierten langkettigen Alkoholen und Chloressigsäure zugänglich sind, eingesetzt werden. Die anionischen Tenside werden vorwiegend in Form ihrer Natriumsalze verwendet.

Besonders geeignete Tenside vom Sulfattyp sind die Schwefelsäuremonoester von primären Alkoholen natürlichen und synthetischen Ursprungs, das heißt, von Fettalkoholen, wie z. B. Kokosfettalkoholen, Talgfettalkoholen, Oleylalkohol oder C₁₀-C₂₀-Oxoalkoholen und solche von sekundären Alkoholen dieser Kettenlängen. Daneben kommen die Schwefelsäuremonoester der mit 1 - 6 Mol Ethylenoxid ethoxilierten aliphatischen primären Alkohole bzw. ethoxilierten sekundären Alkohole bzw. Alkylphenole in Betracht.

Bei den Tensiden vom Sulfonattyp handelt es sich in erster Linie um die Alkylbenzolsulfonate mit C₉₋₁₅-Alkylgruppen und um die Olefinsulfonate, das sind Gemische aus Alken- und Hydroxyalkansulfonaten sowie Disulfonaten, wie man sie beispielsweise aus Monoolefinen mit end- oder innenständiger Doppelbindung durch Sulfonieren mit gasförmigem Schwefeltrioxid und anschließender alkalischer oder saurer Hydrolyse der Sulfonierungsprodukte erhält. Weitere brauchbare Tenside vom Sulfonattyp sind die Alkansulfonate, die aus C₁₂-C₁₈-Alkanen durch Sulfochlorierung oder Sulfoxidation und anschließende Hydrolyse bzw. Neutralisation oder durch Bisulfitaddition an Olefine erhältlich sind, sowie die Ester von alpha-Sulfofettsäuren, z.B. die alpha-sulfonierten Methyl oder Ethylester der hydrierten Kokos-, Palmkern- oder Talgfettsäuren.

Als nichtionische Tenside eignen sich für die erfindungsgemäßen Mittel insbesondere Anlagerungsprodukte von 1 - 100, vorzugsweise 5 - 60 Mol Ethylenoxid (EO) an 1 Mol einer aliphatischen oder alkylaromatischen Verbindung mit im wesentlichen 10 - 20 Kohlenstoffatomen aus der Gruppe der Alkohole, Alkylphenole, Fettsäuren und Fettsäureamide, doch lassen sich auch andere Typen, wie die Ethylenoxidaddukte an Polypropylenoxid und die Kondensationsprodukte aus langkettigen primären Alkoholen und reduzierenden Zuckern, die auch als Alkylglykoside bezeichnet werden, verwenden. Besonders geeignet sind die Anlagerungsprodukte von 20 - 50 Mol Ethylenoxid an primäre Alkohole wie z. B. Kokos- oder Talgfettalkohole, an Oleylalkohol, an Oxoalkohole der entsprechenden Kettenlängen oder an entsprechende sekundäre Alkohole sowie an Mono- oder Dialkylphenole mit 6 - 14 C-Atomen in den Alkylresten.

Als kationische und amphotere Tenside seien Distearyldimethylammoniumchlorid und Alkylbetain genannt.

Die Auswahl der Tenside und ihr Anteil in den einzelnen Massen der erfindungsgemäßen Mittel wird von der beabsichtigten Reinigungsleistung aber auch vom Zusammenwirken mit den anderen Bestandteilen nicht nur bei der Anwendung, sondern auch bei der Herstellung und Lagerung der Mittel bestimmt. Von den oben genannten anionischen Tensiden werden deshalb Alkylbenzolsulfonate, Olefinsulfonate und Fettalkoholsulfate besonders bevorzugt.

Besonders bevorzugte nichtionische Tenside sind die Anlagerungsprodukte von Ethylenoxid an langkettige aliphatische Alkohole mit 12 - 18 C-Atomen und an Alkylphenole mit 6 - 12 C-Atomen in der Alkylgruppe, insbesondere solche Produkte, deren durchschnittlicher Gehalt an Äthylenoxid zwischen 20 und 80 Mol pro Mol Tensid beträgt.

Der Anteil der Tenside in den einzelnen Massen der erfindungsgemäßen Mittel liegt vorzugsweise zwischen etwa 7 und etwa 85 Gew.-%, vorzugsweise zwischen etwa 20 und etwa 60 Gew.-%. Wenn bei den Mitteln auf eine möglichst gleichmäßige Abgabe an Tensiden an das Spülwasser über die gesamte Gebrauchsdauer der Stücke im erfindungsgemäßen Sinne beabsichtigt ist, liegt der Gehalt an Tensiden in der umschließenden Masse vorzugsweise zwischen etwa 15 und etwa 35 Gew.-%, vorzugsweise zwischen etwa 20 und etwa 30 Gew.-%, und liegt in der umschlossenen Masse entsprechend höher, wobei in dieser Masse Tensidkonzentrationen bis etwa 85 Gew.-% ohne weiteres möglich sind. Es ist gerade ein Vorzug der erfindungsgemäß gestalteten Stücke, daß auch hohe Konzentrationen an Wirkstoff in der umschlossenen Masse selbst dann toleriert werden können, wenn sie dieser Masse eine Konsistenz verleihen, die für die Lagerung dieser Masse allein ungeeignet wäre. Durch die Umschließung mit einer Masse von festerer Konsistenz können diese an sich nachteiligen Eigenschaften soweit ausgeglichen werden, daß die Lagerung der Mittel nicht beeinträchtigt ist.

Desinfektionsmittel werden in den erfindungsgemäßen Reinigungsmittelstükken verwendet, wenn neben der Toilettenreinhaltung auch eine keimhemmende Behandlung beabsichtigt wird. Zu diesem Zweck können die erfindungsgemäßen Mittel in einer oder mehrerer der Teilmassen vorzugsweise bis zu etwa 40 Gew.-%, und insbesondere zwischen etwa 0,5 und etwa 10 Gew.-% (jeweils bezogen auf das Gewicht der Teilmassen) an antimikrobiellen Wirkstoffen enthalten. Diese Wirkstoffe können den verschiedensten chemischen Klassen entstammen, doch ist bei der Auswahl, wie auch bei der Auswahl anderer Wirk- und Inhaltsstoffe, auf die Verträglichkeit mit den anderen Komponenten der jeweiligen Massen zu achten. Beispiele geeigneter Wirkstoffklassen sind Phenole, Aktivchlor abspaltende Substanzen und Aktivsauerstoff enthaltende Substanzen. Als Einzelbeispiele aus diesen Klassen seien 1,3-Dichlor-5,5-dimethylhydantoin, Bromchlor-5,5-dimethylhydantoin, 1,3-Dichlor-5-ethyl-5-methylhydantoin, Natriumdichorisocyanurat, Natriumpercarbonat, Natriumperborat (Mono- und Tetrahydrat) und Magnesiummonoperphthalat genannt.

Wenn es bei den Reinigungsmittelstücken auf eine möglichst gleichmäßige Abgabe von antimikrobiellen Wirkstoffen während der gesamten Gebrauchsdauer im erfindungsgemäßen Sinne ankommt, liegt die Konzentration an antimikrobiellen Wirkstoffen in der umschließenden Masse vorzugsweise zwischen etwa 0,5 und etwa 5 Gew.-%, doch ist diese Menge selbstverständlich weitgehend von der Wirksamkeit des jeweiligen antimikrobiellen Wirkstoffes abhängig, so daß in Einzelfällen auch Konzentrationen über oder unter diesen Grenzwerten zweckmäßig sein können. In der umschlossenen Masse liegt die Konzentration an antimikrobiellen Wirkstoffen dann entsprechend höher. Aktivchlor abspaltende Substanzen und Aktivsauerstoff enthaltende Substanzen können in den erfindungsgemäßen Mitteln auch als bleichende Wirkstoffe Verwendung finden.

Als wichtiger Bestandteil zur Untersützung der Reinigungseffektivität, insbesondere gegen Fäkalien- und fetthaltige Ablagerungen innerhalb der Toilette und des Rohrleitungssystems können ein oder mehrere Enzyme, wie Proteasen, Lipasen und Cellulasen, in den erfindungsgemäßen Mitteln enthalten sein. Hierbei sind Lipasen zur Beseitigung fetthaltiger Ablagerungen besonders effektiv. Aktivsauerstoff produzierende Enzyme wie Peroxidasen und Oxidasen können aufgrund ihrer bleichenden und desinfizierenden Eigenschaften besonders vorteilhaft sein. Die Menge an Enzym kann in den erfindungsgemäßen Mitteln in einer oder mehreren Teilmassen bis etwa 60 Gew.-% betragen, sie liegt vorzugsweise zwischen etwa 0,5 und etwa 30 Gew.-%.

Um eine über die Gebrauchsdauer möglichst gleichmäßige Wirksamkeit zu gewährleisten, liegt die Menge an Enyzmen in der umschließenden Masse vorzugsweise etwa zwischen 5 und 15 Gew.-%. Die umschlossene Masse enthält das Enzym in entsprechend höheren Konzentrationen von vorzugsweise 10 bis 30 Gew.-%

Als Wirkstoffe gegen die Wasserhärte, insbesondere aber gegen die in Wässern häufig anzutreffenden Schwermetallionen, wie die des Eisens und des Mangans können die erfindungsgemäßen Mittel Komplexbildner enthalten. Geeignet sind solche Verbindungen, die gegenüber der Wasserhärte und/oder gegenüber diesen Schwermetallionen unter den Anwendungsbedingungen der Mittel eine so starke komplexierende Wirkung entfalten, daß eine Ausfällung von Wasserhärte und/oder eine Ausfällung der Schwermetallhydroxide nicht stattfinden kann. Beispiele geeigneter Komplexbildnerklassen sind Aminopolycarbonsäuren, Polyphosphonsäuren und polymere Polycarbonsäuren und deren Salze, sowie die Salze von Hydroxypolycarbonsäuren. Die einzusetzenden Mengen richten sich nach dem Komplexiervermögen der Komplexbildner und der vorgesehenen Abspülgeschwindigkeit der Reinigungsmittelstücke. Im allgemeinen enthalten die Reinigungsmittelstücke in den einzelnen Massen deshalb nicht mehr als 30 Gew.-% Komplexbildner; meist werden schon mit Mengen zwischen etwa 5 und etwa 25 Gew.-% befriedigende Ergebnisse erzielt. Wenn es auf eine möglichst gleichmäßige Abgabe von Komplexbildner über die gesamte Gebrauchsdauer der Stücke im erfindungsgemäßen Sinne ankommt, liegt die Menge an Komplexbildner in der umschließenden Masse vorzugsweise zwischen etwa 5 und etwa 13 Gew.-%. Die umschlossene Masse enthält entsprechende höhere Konzentrationen.

Zur Verhinderung von Kalksteinablagerungen und vor allem zur Entfernung alter Beläge aus den Toilettenschüsseln können den erfindungsgemäßen Mitteln als Wirkstoffe auch Säuren zugesetzt werden, sofern die Verträglichkeit mit den anderen Komponenten der Mittel gewährleistet ist. Geeignet sind wasserlösliche nichtflüchtige, insbesondere feste Säuren, die einen K_{S}-Wert über 10⁻⁴, vorzugsweise über 10⁻² aufweisen und keine schwerlöslichen Calziumsalze bilden, beispielsweise Amidosulfonsäure, Phosphorsäure, Zitronensäure, Fumarsäure, Apfelsäure, Bernsteinsäure und Glukonsäure. Ihr Anteil an den einzelnen Massen der erfindungsgemäßen Reinigungsmittelstücke kann bis zu 30 Gew.-% betragen und liegt vorzugsweise zwischen etwa 5 und etwa 25 Gew.-%. Wenn es bei den Reinigungsmittelstücken auf eine möglichst gleichmäßige Abgabe dieser Säuren während des gesamten Gebrauchszeitraums im erfindungsgemäßen Sinne ankommt, enthält die umschließende Masse vorzugsweise zwischen etwa 7 und etwa 20 Gew.-%, vorzugsweise zwischen etwa 5 und etwa 10 Gew.-% an Säure. Die umschlossene Masse enthält dann dementsprechend höhere Konzentrationen.

Farbstoffe können den erfindungsgemäßen Mitteln zur optischen Demonstration ihrer Wirksamkeit zugesetzt werden. Es handelt sich dabei vorzugsweise um wasserlösliche Farbstoffe mit meist blauen und grünen Farbtönen. Bei der Auswahl der Farbstoffe ist darauf zu achten, daß sie mit den anderen Bestandteilen der Mittel verträglich sind, aber auch darauf, daß sie gegenüber den keramischen Oberflächen der Toilettenschüsseln nicht substantiv wirken. Beispiele geeigneter Farbstoffe sind Triarylmethanfarbstoffe wie der Triphenylmethanfarbstoff mit der Colour-Index-Nr. 42090.

Die Menge an Farbstoff in den erfindungsgemäßen Mitteln richtet sich nach dem gewünschten Effekt und liegt vorzugsweise nicht über 25 Gew.-%, insbesondere zwischen etwa 3 und etwa 20 Gew.-%, bezogen auf die gesamte Zusammensetzung der farbstoffenthaltenden Teilmasse des Stücks. Wenn es bei den Reinigungsmittelstücken auf eine möglichst gleichmäßige Abgabe von Farbstoffen während der gesamten Gebrauchsdauer ankommt, enthält die umschließende Masse vorzugsweise zwischen etwa 3 und etwa 15 Gew.-%, insbesondere zwischen etwa 3 und etwa 10 Gew.-% an Farbstoff, während die umschlossene Masse entsprechend höhere Konzentrationen dieses Farbstoffs aufweist. Sind die Farbstoffe überwiegend zur Einfärbung der Masse bestimmt, können auch andere Farbstoffe, wie Pigmentfarbstoffe oder öllösliche Farbstoffe verwendet werden, von denen dann im allgemeinen weit geringere Mengen von meist unter 0,1 Gew.-% ausreichen.

Ein besonders bevorzugter Wirkstoff in den erfindungsgemäßen Reinigungsmittelstücken ist Parfüm. Das allmähliche Nachlassen des Dufteindrucks wird neben dem Nachlassen der Farbintensität im Spülwasser bei den herkömmlichen Reinigungsmittelstücken vom Verbraucher als besonders nachteilig empfunden. Demzufolge erscheint die gleichmäßige Abgabe von Duftstoffen bei den erfindungsgemäßen Mitteln als besonders gut wahrnehmbarer Vorteil. Die Wahl der geeigneten Duftstoffe wird im wesentlichen nur durch mögliche Wechselwirkungen mit anderen Bestandteilen der einzelnen Massen der Reinigungsmittelstücke beschränkt. Der Gesamtgehalt an Duftstoffen beträgt in den einzelnen Massen der erfindungsgemäßen Stücke vorzugsweise nicht mehr als 20 Gew.-%; insbesondere liegt er zwischen etwa 4 und etwa 15 Gew.-%. Wenn auf eine möglichst gleichmäßige Abgabe von Duftstoffen während der gesamten Gebrauchsdauer im erfindungsgemäßen Sinne Wert gelegt wird, enthält die umschließende Masse vorzugsweise zwischen etwa 3 und etwa 8 Gew.-%, insbesondere zwischen etwa 4 und etwa 6 Gew.-% an Parfüm, während die umschlossene Masse entsprechend höhere Mengen aufweist. Auch hier wirkt sich der Aufbau der erfindungsgemäßen Stücke vorteilhaft in der Weise aus, daß die umschlossene Masse sehr hohe Konzentrationen an Parfümoel enthalten kann, da ihre Konsistenz für die Lagerstabilität der Blöcke nicht entscheidend ist, sondern letztere wesentlich von der Konsistenz der umschließenden Massen bestimmt wird.

Die als Abspülregulatoren bezeichneten Substanzen dienen in erster Linie dazu, den Verbrauch der Mittel während des Einsatzes so zu steuern, daß die vorgesehene Standzeit eingehalten wird. Als Regulatoren eignen sich vorzugsweise feste langkettige Fettsäuren, wie Stearinsäure, aber auch Salze solcher Fettsäuren, Fettsäureethanolamide, wie Kokosfettsäuremonoethanolamid, Fettalkohole mit vorzugsweise 12 bis 18 C-Atomen oder feste Polyethylenglykole, wie solche mit Molekulargewichten zwischen 1500 und 50000. Im allgemeinen werden nicht mehr als 25 Gew.-% an Abspülregulatoren in den einzelnen Massen benötigt. Bevorzugte Gehaltsbereiche sind etwa 2 Gew.-% bis etwa 15 Gew.-%. Art und Menge der Abspülregulatoren kann in den einzelnen Teilmassen der erfindungsgemäßen Mittel unterschiedlich sein.

Plastifikatoren dienen in erster Linie dazu, den Reinigungsmittelmassen eine für die Formung der Stücke geeignete Plastizität zu verleihen. Es handelt sich dabei um hochsiedende organische Substanzen, die bei der Verarbeitstemperatur flüssig sind. Als Plastifikatoren eignen sich beispielsweise Parafinöle, 1,2-Propylenglykol, Silikonöle, Phthalsäureester, Terpene und Dihydroabietinsäureester. Vorzugsweise werden Dihydroabietinsäuremethylester, Diethylphthalat und Dibutylphthalat eingesetzt. Da die Funktion der Plastifikatoren in vielen Fällen von anderen Bestandteilen der Mittel, vor allem von den Parfümölen, aber auch von nichtionischen Tensiden oder flüssigen Säuren übernommen werden kann, ist ihre Anwesenheit nur in wenigen Fällen notwendig. Sie werden dann in Mengen bis zu 15 Gew.-% eingesetzt.

Durch den Zusatz anorganischer Salze lassen sich ebenfalls die Konsistenz und das Abspülverhalten der Reinigungsmittelstücke beeinflussen, doch verbessert diese Komponente auch die Homogenität der Stücke in kritischen Fällen. Die Salze können weiterhin durch Elektrolyteffekte und als Puffer die Reinigungswirkung der Tenside verstärken; sie können, beispielsweise die Polyphosphate, als härtebindende Stoffe wirken und sie können, wie die Alkalisilikate, als Konsistenzgeber dienen. Als Salze werden im allgemeinen die Alkali- oder Ammoniumsalze von Mineralsäuren, gegebenenfalls in hydratisierter Form, verwendet. Bevorzugt werden die Natriumsalze der Schwefelsäure, der Phosphorsäuren, der Kohlensäure und der Salzsäure. Sie werden in Mengen bis zu etwa 60 Gew.-%, vorzugsweise in Mengen von etwa 10 bis etwa 40 Gew.-% in den einzelnen Massen eingesetzt, wobei auch hier Art und Menge in den einzelnen Teilmassen unterschiedlich sein können.

Die erfindungsgemäßen Mittel können über die bereits erwähnten Inhaltsstoffe hinaus weitere in stückförmigen Toilettenreinigungsmitteln übliche Zusätze enthalten, die das Eigenschaftsbild der Reinigungsmittel abrunden. Als Beispiele seien hier nur Füllstoffe und Konservierungsmittel erwähnt. Füllstoffe, die auch wasserunlöslich sein können, wie beispielsweise Cellulosepulver, fungieren unter anderem als Volumengeber oder als Konsistenzregler und können gegebenenfalls eine Klebrigkeit der Massen beseitigen. Konservierungsmittel haben die Aufgabe, die Reinigungsmittelstücke während der Lagerung und während des Gebrauchs von antimikrobiellem Befall freizuhalten.

Die Anwendung der erfindungsgemäßen Reinigungsmittel unterscheidet sich von der Anwendung der bisher für diese Zwecke bekannten stückförmigen Mittel nicht. Stücke, die für den Einsatz im Wasserkasten der Toilette bestimmt sind, werden dort entweder ohne weitere Hilfsmittel eingeworfen, oder aber in käfigartigen Behältern dort so angebracht, daß sie vom zulaufenden oder vom gespeicherten Wasser erreicht werden. Mittel, die für die Anwendung in der Toilettenschüssel bestimmt sind, werden an geeigneten Haltern oder in korb- oder käfigartigen Behältern im Toilettenbecken an einer Stelle angebracht, die bei jedem Spülvorgang vom zulaufenden Spülwasser durchströmt wird. Üblicherweise werden sie unter dem Innenrand des Toilettenbeckens eingehängt.

### Beispiele

In den folgenden Beispielen ist jeweils zunächst die Zusammensetzung der einzelnen Massen, aus denen die Stücke bestehen, aufgeführt. Soweit nicht anders angegeben, bedeuten die Zahlenangaben hier Gewichtsprozent; GT bedeutet Gewichtsteile. Im Anschluß daran sind die Gewichte der Reinigungsmittelstücke sowie die Gewichte der Teilmassen, aus denen sie bestehen, aufgeführt. Dann folgen Angaben zur Herstellung und Prüfung der Eigenschaften.

### Beispiel 1: WC-Stick mit flüssigem Desinfektionsmittel

| Rohstoff | Mantel | Kern |
|---|---|---|
| Natriumlaurylsulfat | 12,0 % | 12,0 % |
| Talgalkohol + 25 EO | 28,0 % | 28,0 % |
| Kokosfettsäuremonoethanolamid | 5,0 % | 5,0 % |
| Cellulosepulver | 6,0 % | 6,0 % |
| Na-citrat (Dihydrat) | 5,0 % | 5,0 % |
| Na₂SO₄ | 34,0 % | 32,0 % |
| Na₂CO₃ | 2,0 % | 2,0 % |
| Parfümöl | 4,0 % | 4,0 % |
| Palmitin-Stearinsäuregemisch | 2,0 % | 2,0 % |
| (1 : 1) | | |
| Flüssiges Desinfektionsmittel | 2,0 % | 4,0 % |
| Bodoxin (Bode-Chemie) (Wz) | | |
| | | |
| Gesamtgewicht 50 g: | Mantel 25 g | Kern 25 g |

### Verfahren zur Herstellung:

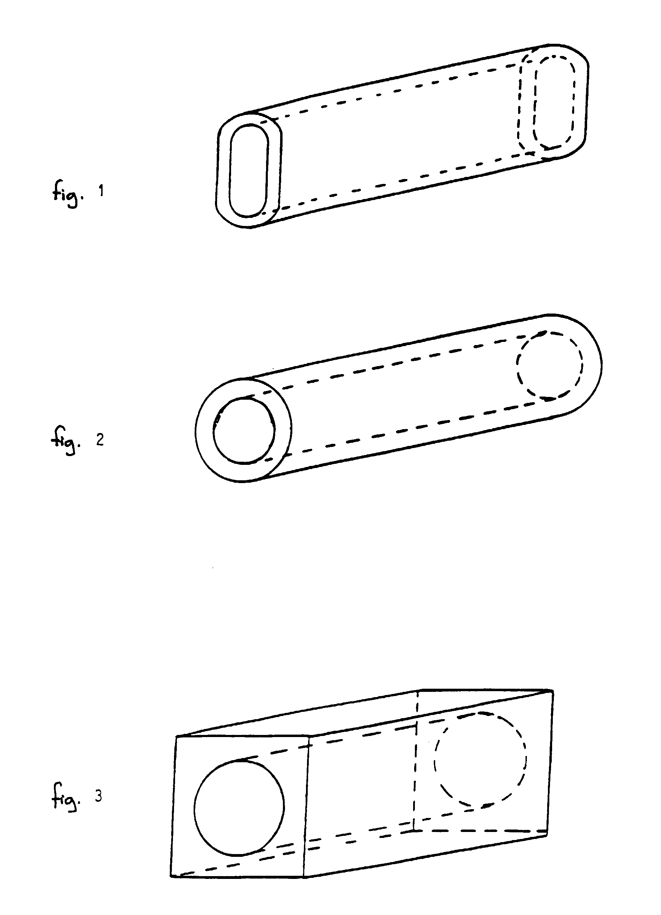

Die pulverförmigen Rohstoffe von Mantel und Kern wurden in 2 separaten Mischern vorgemischt und in 2 Silos bevorratet. Die Dosierung beider Mischungen in die Extruder 1 (Mantel) und 2 (Kern) erfolgte über 2 Bandwaagen. Die Zugabe der unterschiedlichen Desinfektionsmittelanteile in die Extruder 1 und 2 wurde als Mischung mit Parfümöl über Dosierpumpem vorgenommen. In einer Mehrstoffdüse wurden die Stränge im Gewichtsverhältnis 1 : 1 zusammengeführt und mit einem automatischen Schneidegerät auf die gewünschte Länge geschnitten. Der Stick hatte eine Form gemäß Abbildung 1 mit Außenabmessungen von etwa 15 x 27 x 86 mm.

### Beispiel 2: WC-Stick mit pulverförmigem Desinfektionsmittel

| Rohstoff | Mantel | Kern |
|---|---|---|
| Natriumlaurylsulfat | 9,0 % | 9,0 % |
| Talgalkohol + 25 EO | 28,0 % | 28,0 % |
| Kokosfettsäuremonoethanolamid | 9,0 % | 9,0 % |
| Cellulosepulver | 6,0 % | 6,0 % |
| Na-citrat (Dihydrat) | 5,0 % | 5,0 % |
| Na₂SO₄ | 35,0 % | 33,5 % |
| Na₂CO₃ | 1,5 % | 1,5 % |
| Parfümöl | 5,0 % | 5,0 % |
| Diazolindinyl Harnstoff | 1,5 % | 3,0 % |
| (Germall II) (Wz) | | |
| | | |
| Gesamtgewicht 50 g: | Mantel 25 g | Kern 25 g |

### Verfahren zur Herstellung:

Die Herstellung erfolgte wie unter Beispiel 1 beschrieben. Es wurde aber das pulverförmige Desinfektionsmittel in den entsprechenden Konzentrationen den separaten Mischern zugegeben und mit den übrigen Pulverrohstoffen homogen vermischt. Es wurden Rundsticks von etwa 24 mm Durchmesser und etwa 80 mm Länge (Abbildung 2) mit 50 g Gesamtgewicht und einer Aufteilung von Mantel und Kern zu je 25 g geschnittenen und in wasserlöslicher Folie verpackt.

### Beispiel 3: Wasserkastenwürfel mit Desinfektionswirkung

| Rohstoff | Mantel | Kern |
|---|---|---|
| Natriumdodecylbenzolsulfonat (Ufaryl (Wz) DL 80 CW; 80 % DBS, 13 % Na₂SO₄, 5 % Na-Citrat, 2 % Free Oil) | 47,5 % | 44,0 % |
| Na₂SO₄ | 15,0 % | - % |
| Xanthan (Rhodopol 50 MD) (Wz) | 2,0 % | 4,0 % |
| Dyhydroabietinsäuremethylester (Hercolyn D) (Wz) | 12,0 % | 12,0 % |
| Na-dichlorisocyanurat-Dihydrat | 23,5 % | 40,0 % |
| | | |
| Gesamtgewicht 50 g: | Mantel 25 g | Kern 25 g |

### Verfahren zur Herstellung:

Die Rohstoffkomponenten wurden jeweils in einem separaten Z-Kneter unter Zusatz des Plastifikators (Hercolyn D) in einer Chargengröße von je 150 kg gemischt und plastifiziert. Über Austragsschnecken wurden die beiden Komponenten in die entsprechende Formpresse befördert und über ein 2fach-Mundstück zu einem Würfel von etwa 34 mm Kantenlänge vereint (Abbildung 7).

Die Haltbarkeit der 50 g-Würfel betrug bei einer Abspülrate von 21 Spülungen/Tag etwa 400 Spülungen. Bei stündlicher Spülung war ein für die Desinfektion ausreichender Aktivchlorgehalt von 0,3 - 1,0 ppm in Spülwasser nachweisbar.

### Beispiel 4: WC-Stick mit höherem Duftanteil im gegossenen Kern

| Rohstoff | Mantel | Kern |
|---|---|---|
| Natriumlaurylsulfat | 14,0 % | - % |
| Kokosfettsäuremonoethanolamid | 3,0 % | - % |
| Talgalkohol + 25 EO | 21,0 % | 65,0 % |
| Cellulosepulver | 8,0 % | - % |
| Na-citrat (Dihydrat) | 7,5 % | - % |
| Na₂SO₄ | 36,0 % | - % |
| Na₂CO₃ | 2,5 % | - % |
| Parfümöl | 5,0 % | 15,0 % |
| Palmitin-Stearinsäuregemisch | 3,0 % | 5,0 % |
| (1 : 1) | | |
| Polyethylenglykol 1500 | - % | 15,0 % |
| | | |
| Gesamtgewicht 50 g: | Mantel 35 g | Kern 15 g |

### Verfahren zur Herstellung:

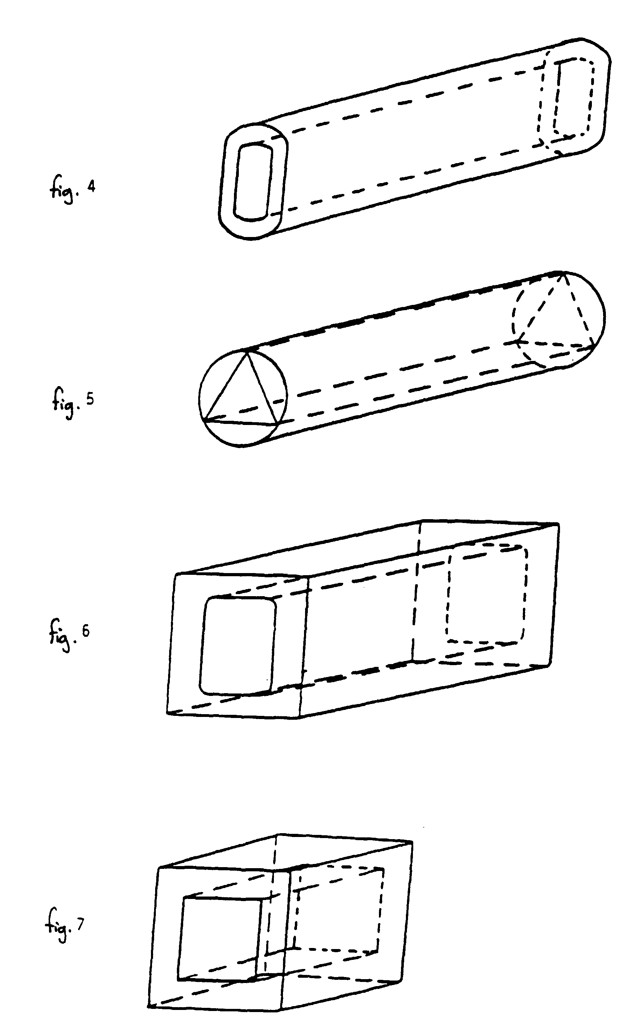

Nach Vormischung der Rohstoffe und Extrusion des Hohlmantels wurde dieser auf die endgültige Länge zugeschnitten und so auf einer Unterlage placiert, daß das Formteil unten verschlossen war. Die Rohstoffkomponenten der Kern-Rezeptur wurden in einem beheizbaren Kessel aufgeschmolzen und unter Rühren mit dem Parfümöl vermischt. Mit hilfe eines Gießwagens wurden die Hohlmäntel befüllt und durch einen Kühlkanal geleitet. Die abgekühlten Muster wurden in ein entsprechendes Behältnis verpackt und mit Cellglas duftdicht umschlossen. Der Stick gemäß Abbildung 4 hatte Außenabmessungen von etwa 15 x 27 x 88 mm.

Unter Standardspülbedingungen wurden ca. 280 Spülungen erzielt.

Vorteil dieser Ausführungsform ist die Möglichkeit zur Einarbeitung sehr hoher Parfümdosierungen im Kern. Über die gesamte Anwendungsdauer wurde ein besonders gleichmäßiger Dufteindruck erzielt.

### Beispiel 5: Extrudierter 2-Phasenstick mit Duftintensivkern

| Rohstoff | Mantel | Kern |
|---|---|---|
| Natriumlaurylsulfat | 12,0 GT | |
| Talgalkohol + 25 EO | 28,0 GT | |
| Kokosfettsäuremonoethanolamid | 3,0 GT | |
| Cellulosepulver | 6,0 GT | gleiche |
| Na-citrat (Dihydrat) | 5,0 GT | Grund- |
| Na₂SO₄ | 36,0 GT | mischung |
| Na₂CO₃ | 2,0 GT | |
| Palmitin-Stearinsäuregemisch | 3,0 GT | |
| (1 : 1) | | |
| Parfümöl Citrus-Note | 5,0 GT | 9,0 GT |
| | | |
| Gesamtgewicht 50 g: | Mantel 25 g | Kern 25 g |

### Verfahren zur Herstellung:

Masse und Kern wurden aus der gleichen Grundmischung erstellt. Hierzu wurden die pulverförmigen Rohstoffe in einem Mischer zusammengeführt und in ein Vorratssilo überführt. Die Dosierung erfolgte über eine Bandwaage mit Mengenteiler in 2 gesonderte Extruder. In jeden Extruder wurde der unterschiedliche Parfümanteil über Dosierpumpen eingedüst. Über einen speziellen Doppelkopf wurden mittels einer Bepex-Formpresse die 2-Phasenstränge ausgetragen und mit einem pneumatischen Schneider formgerecht getrennt. Die Sticks gemäß Abbildung 2 hatten einen äußeren Durchmesser von etwa 24 mm und eine Länge von etwa 80 mm.

Dieses Verfahren ist besonders vorteilhaft, da ein 2. Mischaggregat mit Vorratsbehälter und Bandwaage nicht erforderlich ist. Im Standardtest erreichte dieser Stick eine Haltbarkeit von 250 Spülungen.

### Beispiel 6: Extrudierter 2-Phasenstick mit Duftintensivkern

| Rohstoff | Mantel | Kern |
|---|---|---|
| Natriumlaurylsulfat | 14,0 GT | |
| Talgalkohol + 25 EO | 19,0 GT | |
| Kokosfettsäuremonoethanolamid | 6,0 GT | |
| Cellulosepulver | 8,0 GT | gleiche |
| Na-citrat (Dihydrat) | 7,5 GT | Grund- |
| Na₂SO₄ | 34,55 GT | mischung |
| Na₂CO₃ | 0,95 GT | |
| Myristylalkohol (Lorol-C₁₄) | 5,0 GT | |
| Parfümöl blumige Note | 5,0 GT | 11,0 GT |
| | | |
| Gesamtgewicht 50 g: | Mantel 32 g | Kern 18 g |

Das Verfahren zur Herstellung entsprach dem in Beispiel 5, doch wurde hier eine Form gemäß Abbildung 5 (etwa 24 x 80 mm) gewählt. Diese Ausführungsform ist besonders vorteilhaft, da mit zunehmenden Verbrauch der höher parfümierte Intensivkern kontinuierlich freigespült wurde. Die Haltbarkeit der Stücke betrug 320 Spülungen unter Standardbedingungen.

### Beispiel 7: Extrudierter 2-Phasenstick mit Duftintensivkern

| Rohstoff | Mantel | Kern |
|---|---|---|
| Natriumlaurylsulfat | 15,0 GT | |
| Talgalkohol + 25 EO | 15,0 GT | |
| Kokosfettsäuremonoethanolamid | 6,0 GT | |
| Cellulosepulver | 3,0 GT | gleiche |
| Na-citrat (Dihydrat) | 3,5 GT | Grund- |
| Na₂SO₄ | 36,5 GT | mischung |
| Na₂CO₃ | 2,0 GT | |
| Natriumdodecylbenzolsulfonat | 15,0 GT | |
| Parfümöl Apfel-Note | 4,0 GT | 6,0 GT |
| | | |
| Gesamtgewicht 50 g: | Mantel 25 g | Kern 25 g |

Das Verfahren zur Herstellung entsprach dem in Beispiel 5, wobei hier eine Form gemäß Abbildung 1 (etwa 15 x 27 x 86 mm) gewählt wurde.

Die Haltbarkeit im Standardtest betrug 300 Spülungen.

### Beispiel 8: Wasserkastenblöcke

| Rohstoff | Mantel | Kern |
|---|---|---|
| Natriumlaurylsulfat | 19,0 % | 19,0 % |
| Kokosfettsäuremonoethanolamid | 33,0 % | 33,0 % |
| Na-citrat (Dihydrat) | 5,0 % | 5,0 % |
| Na₂SO₄ | 18,0 % | 14,0 % |
| NA₂CO₃ | 2,0 % | 2,0 % |
| Acid Blue 9 | 15,0 % | 15,0 % |
| Parfümöl blumige Note | 8,0 % | 12,0 % |
| | | |
| Gesamtgewicht 100 g: | Mantel 50 g | Kern 50 g |

Das Verfahren zur Herstellung entsprach dem in Beispiel 3. Es wurden Blöcke sowohl nach Abbildung 3 als auch nach Abbildung 6, jeweils mit den Außenabmessungen von etwa 34 x 34 x 68 mm, hergestellt. In beiden Fällen lag die Haltbarkeit im Standardtest bei etwa 1400 Spülungen. Bei beiden Blockformen wurde der Duft sehr gleichmäßig abgegeben.

### Beispiel 9: Wasserkastenblöcke

| Rohstoff | Mantel | Kern |
|---|---|---|
| Natriumlaurylsulfat | 11,4 % | 11,4 % |
| Kokosfettsäuremonoethanolamid | 8,5 % | 8,5 % |
| Na-citrat (Dihydrat) | 9,0 % | 18,5 % |
| Na₂SO₄ | 58,3 % | 47,8 % |
| NA₂CO₃ | 0,8 % | 0,8 % |
| Acid Blue 9 | 4,0 % | 4,0 % |
| Natriumstearat | 2,0 % | 3,0 % |
| Parfümöl Fichten-Note | 6,0 % | 6,0 % |
| Gesamtgewicht 100 g: | Mantel 60 g | Kern 40 g |

Das Verfahren zur Herstellung entsprach dem in Beispiel 3. Es wurden Blöcke sowohl nach Abbildung 3 als auch nach Abbildung 6 mit Außenabmessungen von jeweils etwa 32 x 32 x 66 mm ausgeformt. In beiden Fällen lag die Haltbarkeit im Standardtest bei etwa 1100 Spülungen. Die prophylaktische Wirkung gegen Kalkablagerungen blieb über die gesamte Anwendungsdauer weitgehend konstant.

### Beispiel 10: Wasserkastenblöcke

| Rohstoff | Mantel | Kern |
|---|---|---|
| Natriumlaurylsulfat | 19,0 % | 19,0 % |
| Kokosfettsäuremonoethanolamid | 35,0 % | 35,0 % |
| Na₂SO₄ | 10,0 % | - % |
| NA₂CO₃ | 2,5 % | 2,5 % |
| Acid Blue 9 | 15,0 % | 15,0 % |
| Acrylsäure-Maleinsäure-Co-polymerisat | 12,5 % | 22,5 % |
| (Sokalan CP 5) (Wz) | | |
| Parfümöl Fichten-Note | 6,0 % | 6,0 % |
| | | |
| Gesamtgewicht 100 g: | Mantel 50 g | Kern 50 g |

Das Verfahren zur Herstellung entsprach dem in Beispiel 3. Es wurden Blöcke sowohl nach Abbildung 3 als auch nach Abbildung 6 mit Außenabmessungen von jeweils etwa 34 x 34 x 68 mm ausgeformt. In beiden Fällen lag die Haltbarkeit im Standardtest bei etwa 550 Spülungen. Die prophylaktische Wirkung gegen Kalkablagerungen blieb über die gesamte Anwendungsdauer weitgehend konstant.

### Beispiel 11: Wasserkastenblöcke

| Rohstoff | Mantel | Kern |
|---|---|---|
| Natriumlaurylsulfat | 15,0 % | 15,0 % |
| Kokosfettsäuremonoethanolamid | 35,0 % | 35,0 % |
| Na₂SO₄ | 13,5 % | - % |
| Natriumstearat | 8,0 % | 7,0 % |
| Acid Blue 9 | 7,5 % | 15,0 % |
| Zitronensäure | 15,0 % | 25,0 % |
| Parfümöl Wintergrün | 6,0 % | 3,0 % |
| Gesamtgewicht 100g: | Mantel 50 g | Kern 50 g |

Das Verfahren zur Herstellung entsprach dem in Beispiel 3. Es wurden Blöcke sowohl nach Abbildung 3 als auch nach Abbildung 6 mit Außenabmessungen von jeweils etwa 34 x 34 x 68 mm ausgeformt. In beiden Fällen lag die Haltbarkeit im Standardtest bei etwa 1600 Spülungen. Die prophylaktische Wirkung gegen Kalkablagerungen blieb über die gesamte Anwendungsdauer weitgehend konstant.

### Beispiel 12:

| Rohstoff | Mantel | Kern |
|---|---|---|
| Natriumlaurylsulfat | 11,2 % | 11,2 % |
| Kokosfettsäuremonoethanolamid | 8,5 % | 8,5 % |
| Na₂SO₄ | 57,5 % | 54,5 % |
| Natriumstearat | 3,0 % | 3,0 % |
| Na₂CO₃ | 0,8 % | 0,8 % |
| Acid Blue 9 | 4,0 % | 7,0 % |
| Na-citrat (Dihydrat) | 9,0 % | 9,0 % |
| Parfümöl Citrus | 6,0 % | 6,0 % |
| | | |
| Gesamtgewicht 100 g: | Mantel 50 g | Kern 50 g |

Das Verfahren zur Herstellung entsprach dem in Beispiel 3. Es wurden Blöcke sowohl nach Abbildung 3 als auch nach Abbildung 6 mit Außenabmessungen von jeweils etwa 32 x 32 x 66 mm ausgeformt. In beiden Fällen lag die Haltbarkeit im Standardtest bei etwa 1000 Spülungen. Die Anfärbung des Toilettenspülwassers blieb über die gesamte Anwendungsdauer weitgehend konstant.

### Beispiel 13: Extrudierter 2-Phasenstick mit Kalkprophylaxe-Intensivkern

| Rohstoff | Mantel | Kern |
|---|---|---|
| Natriumlaurylsulfat | 14,0 % | 14,0 % |
| Talgalkohol + 25 EO | 21,5 % | 16,5 % |
| Kokosfettsäuremonoethanolamid | 3,0 % | 3,0 % |
| Cellulosepulver | 8,0 % | 8,0 % |
| Na-citrat (Dihydrat) | 5,0 % | 10,0 % |
| Na₂SO₄ | 37,0 % | 37,0 % |
| Na₂CO₃ | 2,5 % | 2,5 % |
| Palmitin-Stearinsäuregemisch | 3,0 % | 3,0 % |
| (1 : 1) | | |
| Parfümöl | 6,0 % | 6,0 % |
| | | |
| Gesamtgewicht 50 g: | Mantel 30 g | Kern 20 g |

Das Verfahren zur Herstellung entsprach dem in Beispiel 1. Die Form wurde gemäß Abbildung 4 gewählt mit Außenabmessungen von etwa 15 x 27 x 86 mm. Im Standardtest betrug die Haltbarkeit 300 Spülungen bei sehr gleichmäßiger Prophylaxewirkung.

### Beispiel 14: Extrudierter 2-Phasenstick mit Kalkprophylaxe-Intensivkern

| Rohstoff | Mantel | Kern |
|---|---|---|
| Natriumlaurylsulfat | 14,0 % | 14,0 % |
| Talgalkohol + 25 EO | 19,0 % | 17,0 % |
| Kokosfettsäuremonoethanolamid | 3,0 % | 3,0 % |
| Cellulosepulver | 8,0 % | 8,0 % |
| Na₂SO₄ | 36,5 % | 34,0 % |
| Na₂CO₃ | 2,0 % | 2,0 % |
| Myristylalkohol | 4,0 % | 4,0 % |
| Natriumgluconat | 7,5 % | 12,0 % |
| Parfümöl | 6,0 % | 6,0 % |
| | | |
| Gesamtgewicht 50 g: | Mantel 30 g | Kern 20 g |

Das Verfahren zur Herstellung entsprach dem in Beispiel 1. Die Form wurde gemäß Abbildung 5 gewählt (etwa 24 x 80 mm). Im Standardtest betrug die Haltbarkeit 260 Spülungen bei sehr gleichmäßiger Prophylaxewirkung.

### Beispiel 15: Extrudierter 2-Phasenstick mit Kalkprophylaxe-Intensivkern

| Rohstoff | Mantel | Kern |
|---|---|---|
| Natriumlaurylsulfat | 14,0 % | 14,0 % |
| Talgalkohol + 25 EO | 19,0 % | 19,0 % |
| Kokosfettsäuremonoethanolamid | 3,0 % | 3,0 % |
| Cellulosepulver | 8,0 % | 8,0 % |
| Na₂SO₄ | 37,5 % | 33,0 % |
| Myristylalkohol | 5,0 % | 5,0 % |
| Fumarsäure | 7,5 % | 12,0 % |
| Parfümöl | 6,0 % | 6,0 % |
| | | |
| Gesamtgewicht 50 g: | Mantel 25 g | Kern 25 g |

Das Verfahren zur Herstellung entsprach dem in Beispiel 1. Die Form wurde gemäß Abbildung 1 mit Außenabmessungen von 15 x 27 x 86 mm gewählt. Im Standardtest betrug die Haltbarkeit 250 Spülungen bei sehr gleichmäßiger Prophylaxewirkung.

### Beispiel 16: Extrudierter 2-Phasenstick mit Kalkprophylaxe-Intensivkern

| Rohstoff | Mantel | Kern |
|---|---|---|
| Natriumlaurylsulfat | 18,0 % | 18,0 % |
| Talgalkohol + 25 EO | 20,0 % | 20,0 % |
| Kokosfettsäuremonoethanolamid | 3,0 % | 3,0 % |
| Cellulosepulver | 20,0 % | 15,0 % |
| Na₂SO₄ | 23,0 % | 23,0 % |
| Palmitin-Stearinsäuregemisch | 2,0 % | 2,0 % |
| (1 : 1) | | |
| Zitronensäure | 10,0 % | 15,0 % |
| Parfümöl | 4,0 % | 4,0 % |
| | | |
| Gesamtgewicht 50 g: | Mantel 25 g | Kern 25 g |

Das Verfahren zur Herstellung entsprach dem in Beispiel 1. Die Form wurde gemäß Abbildung 2 (etwa 24 x 86 mm) gewählt. Im Standardtest betrug die Haltbarkeit 300 Spülungen bei sehr gleichmäßiger Prophylaxewirkung.

### Beispiel 17: WC-Stick mit 2 Phasen und Sperrschicht

| Rohstoff | Mantel | Kern 1 | Kern 2 |
|---|---|---|---|
| Natriumlaurylsulfat | 12,0 GT | | |
| Talgalkohol + 25 EO | 28,0 GT | 60,0 GT | |
| Kokosfettsäuremonoethanolamid | 3,0 GT | | |
| Cellulosepulver | 6,0 GT | | gleiche |
| Na-citrat (Dihydrat) | 5,0 GT | | Grund- |
| Na₂SO₄ | 35,0 GT | | mischung |
| Na₂CO₃ | 2,0 GT | | wie |
| Palmitin-Stearinsäuregemisch | 4,0 GT | | Mantel |
| (1 : 1) | | | |
| Polyethylenglykol 1500 | | 40,0 GT | |
| Parfümöl Citrus-Note | 5,0 GT | | 9,0 GT |
| | | | |
| Gesamtgewicht 50 g: | 25,0 g | 5,0 g | 20,0 g |

### Verfahren zur Herstellung:

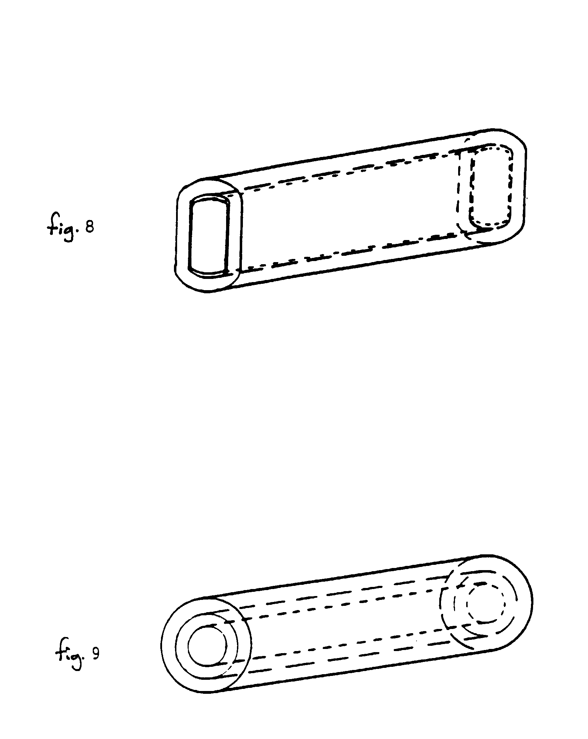

Masse und Kern 2 wurden aus der gleichen Grundmischung erstellt. Die pulverförmigen Rohstoffe werden in einem Mischer zusammengeführt und in ein Vorratssilo überführt. Die Rohstoffe von Kern 1 wurden in einem beheizten Kessel aufgeschmolzen. Die Extrusion erfolgt in einer 3fach Bepex-Formpresse in der Weise, daß die geschmolzene Masse als Sperrschicht in 1 mm Stärke zwischen Mantel und Kern 2 zugeführt wurde. Der Stick gemäß Abbildung 8 hatte Außenabmessungen von etwa 15 x 27 x 86 mm.

Durch die Zwischenschicht wurde die Auswanderung des Parfüms vom Kern 2 in dem Mantel gebremst. Die Haltbarkeit betrug im Standardtest 280 Spülungen.

### Beispiel 18: Extrudierter 3-Phasenstick mit Duftintensivkern

| Rohstoff | Mantel | Kern 1 | Kern 2 |
|---|---|---|---|
| Natriumlaurylsulfat | 12,0 GT | | |
| Talgalkohol + 25 EO | 28,0 GT | | |
| Kokosfettsäuremonoethanolamid | 3,0 GT | | |
| Cellulosepulver | 6,0 GT | gleiche Grund mischung wie Mantel | |
| Na-citrat (Dihydrat) | 5,0 GT | | |
| Na₂SO₄ | 36,0 GT | | |
| Na₂CO₃ | 2,0 GT | | |
| Palmitin-Stearinsäuregemisch | 4,0 GT | | |
| (1 : 1) | | | |
| Parfümöl Citrus-Note | 4,0 GT | 8,0 GT | 10,0 GT |
| | | | |
| Gesamtgewicht 50 g: | 20,0 g | 20,0 g | 10,0 g |

### Verfahren zur Herstellung:

Es wurde eine Grundmischung verwendet. Die Dosierung der einzelnen Phasen erfolgt über Bandwaagen in 3 gesonderte Extruder. In jeden Extruder wurde die Parfümierung über Dosierpumpen eingedüst. In einer Bepex-Formpresse mit einem 3fach-Kopf wurden die Stränge ausgetragen und anschließend formgerecht geschnitten. Die Form entsprach Abbildung 9 mit Außenabmessungen von etwa 24 x 80 mm.

Die Sticks zeigten im Standardtest eine Haltbarkeit von 300 Spülungen bei ganz besonders gleichmäßiger Duftabgabe.

### Beispiel 19: WC-Stick mit Lipase

| Rohstoff | Mantel | Kern |
|---|---|---|
| Natriumlaurylsulfat | 12,6 % | 12,6 % |
| Talgalkohol + 25 EO | 16,0 % | 16,0 % |
| Kokosfettsäuremonoethanolamid | 6,0 % | 6,0 % |
| Alkylbenzolsulfonat-Na | 12,0 % | 12,0 % |
| Cellulosepulver | 3,0 % | 3,0 % |
| Na-citrat (Dihydrat) | 3,5 % | 3,5 % |
| Na₂SO₄ | 24,9 % | 14,9 % |
| Na₂CO₃ | 2,0 % | 2,0 % |
| Parfümöl | 6,0 % | 6,0 % |
| C₁₆-Fettalkohol | 4,0 % | 4,0 % |
| Lipase* | 10,0 % | 20,0 % |
| | | |
| Gesamtgewicht 50 g: | Mantel 25 g | Kern 25 g |

| | | |
|---|---|---|
| *) Fa. Gist-Brocades BSD BV, Netherlands | | |

Die pulverförmigen Rohstoffe von Mantel und Kern wurden in 2 separaten Mischern gemischt. Danach erfolgt eine Nachmischung mit Lipase. Die Bevorratung erfolgt in 2 Silos. Die Dosierung beider Mischungen in die Extruder 1 (Mantel) und 2 (Kern) erfolgte über 2 Bandwaagen, wobei zur Plastifizierung in den Extrudern 1 und 2 Parfümöl über Dosierpumpen zugesetzt wurde. In der Mehrstoffdüse wurden die Stränge im Gewichtsverhältnis 1:1 zusammengefügt und auf die gewünschte Länge geschnitten. Die Form entsprach Abbildung 1 mit Abmessungen von etwa 16 x 28 x 80 mm.

### Beispiel 20: Wasserkastenblock mit Enzymen

| Rohstoff | Mantel | Kern |
|---|---|---|
| Natriumlaurylsulfat | 11,4 % | 11,4 % |
| Kokosfettsäuremonoethanolamid | 8,5 % | 8,5 % |
| Na-citrat (Dihydrat) | 9,0 % | 18,5 % |
| Na₂SO₄ | 48,3 % | 29,8 % |
| Na₂CO₃ | 0,8 % | 0,8 % |
| Acid Blue 9 | 4,0 % | 4,0 % |
| Natriumstearat | 2,0 % | 3,0 % |
| Parfümöl Fichten-Note | 6,0 % | 6,0 % |
| Protease* | 5,0 % | 8,0 % |
| Lipase* | 5,0 % | 10,0 % |
| | | |
| Gesamtgewicht 100 g: | Mantel 60 g | Kern 40 g |

| | | |
|---|---|---|
| *) Fa. Gist-Brocades BSD BV, Netherlands | | |

Die Rohstoffe, mit Ausnahme der Enzyme, wurden jeweils in separaten Z-Knetern unter Zusatz des Parfüms zu einer Chargengröße von 300 bzw. 200 kg gemischt und verknetet. Anschließend erfolgte die Zugabe der Enyzme und eine kurze Nachmischung. Über 2 Extruder wurden die beiden Komponenten in eine Formpresse befördert, über ein 2fach-Mundstück extrudiert und zu Würfeln von etwa 34 mm Kantenlänge geschnitten (Abb. 6).

## Patentansprüche

1. Reinigungsmittel in Stückform für Spültoiletten, das aus wenigstens zwei unterschiedlich zusammengesetzten Massen besteht, wobei eine der Massen von der oder den anderen Massen wenigstens teilweise umschlossen ist, dadurch gekennzeichnet, daß die umschlossene Masse und wenigstens eine der anderen Massen wenigstens einen gleichen Wirkstoff enthalten, wobei die Konzentration wenigstens eines dieser Wirkstoffe in der umschlossenen Masse wenigstens das 1,3fache der Konzentration des gleichen Wirkstoffs in der oder den umschließenden Massen beträgt und der Gehalt an Salzen langkettiger Fettsäuren in den Massen nicht mehr als 25 Gew.-% beträgt.

2. Reinigungsmittel nach Anspruch 1, bei dem die Konzentration wenigstens eines der Wirkstoffe, die sowohl in der umschlossenen als auch in wenigstens einer der umschließenden Massen enthalten sind, in der umschlossenen Masse 2 bis 10 mal so hoch ist wie in einer der umschließenden Massen.

3. Reinigungsmittel nach einem der Ansprüche 1 oder 2, bei dem die umschlossene Masse auf wenigstens 70 %, vorzugsweise wenigstens 80 % ihrer Oberfläche von den anderen Massen bedeckt ist.

4. Reinigungsmittel nach einem der Ansprüche 1 bis 3, dadurch gekennzeichnet, daß die umschlossene Masse nur von einer weiteren Masse umschlossen ist.

5. Reinigungsmittel nach einem der Ansprüche 1 bis 4, bei dem wenigstens einer der Wirkstoffe, der in der umschlossenen Masse in der geforderten höheren Konzentration als in der umschließenden Masse enthalten ist, aus der Gruppe Parfüm, Komplexbildner, Säuren, Enzym, Desinfektions- und Bleichmittel ausgewählt ist.

6. Reinigungsmittel nach Anspruch 5, bei dem der Wirkstoff, der in der umschlossenen Masse in der geforderten höheren Konzentration als in der umschließenden Masse enthalten ist, Parfüm ist.

## Claims

1. A block-form cleaner for flush toilets consisting of at least two masses of different composition, one of the masses being at least partly surrounded by the other mass(es), characterized in that the surrounded mass and at least one of the other masses contain at least one identical active substance, the concentration of at least one of these active substances in the surrounded mass being at least 1.3 times.the concentration of the same active substance in the surrounding mass(es) and the content of salts of long-chain fatty acids in the masses being no more than 25% by weight.

2. A toilet cleaner as claimed in claim 1, in which the concentration of at least one of the active substances, which is present both in the surrounded mass and in at least one of the surrounding masses, in the surrounded mass is 2 to 10 times higher than in one of the surrounding masses.

3. A toilet cleaner as claimed in claim 1 or 2, in which at least 70% and preferably at least 80% of the surface of the surrounded mass is covered by the other masses.

4. A toilet cleaner as claimed in any of claims 1 to 3, characterized in that the surrounded mass is surrounded by only one other mass.

5. A toilet cleaner as claimed in at least one of claims 1 to 4, in which at least one of the active substances which is present in the surrounded mass in the required higher concentration than in the surrounding mass is selected from the group consisting of perfume, complexing agents, acids, enzyme, disinfectants and bleaching agents.

6. A toilet cleaner as claimed in claim 5, in which the active substance which is present in the surrounded mass in the required higher concentration than in the surrounding mass is perfume.

## Revendications

1. Agent de nettoyage en morceaux ou batonnets pour toilettes à chasse d'eau qui se compose d'au moins deux masses réunies différentes, une des masses étant entourée au moins partiellement par la ou les autres masses,
caractérisé en ce que
la masse enfermée et au moins une des autres masses contiennent au moins une même matière active, la concentration d'au moins une de ces matières actives se montant dans la masse enfermée au moins à 1,3 fois la concentration de la même matière active dans la ou les masses qui enferment et la teneur en sels d'acides gras à longue chaîne dans les masses ne dépassant pas 25 % en poids.

2. Agent de nettoyage selon la revendication 1,
dans lequel
la concentration d'au moins une des matières actives qui sont contenues aussi bien dans les masses enfermée que dans au moins une des masses qui enferment, est dans la masse enfermée 2 à 10 fois aussi élevée que dans une des masses qui enferment.

3. Agent de nettoyage selon une des revendications 1 ou 2,
dans lequel
la masse enfermée est recouverte au moins à 70 %, de préférence au moins à 80 % de leur surface par les autres masses.

4. Agent de nettoyage selon une des revendications 1 à 3,
caractérisé en ce que
la masse enfermée n'est enfermée que par une autre masse.

5. Agent de nettoyage selon une des revendications 1 à 4,
dans lequel
au moins une des matières actives qui est contenue dans la masse enfermée dans la concentration plus élevée exigée que dans la masse qui enferme, est choisie dans le groupe des parfums, compléments, acides, enzymes, agents de désinfection et de blanchiment.

6. Agent de nettoyage selon la revendication 5,
dans lequel
la matière active qui est contenue dans la masse enfermée dans la concentration plus élevée exigée que dans la masse qui entoure, est du parfum.
